# EUROPEAN PATENT APPLICATION

(11) **EP 2 777 732 A1**
(43) Date of publication of application: **17.09.2014**
(21) Application number: 11875316.9
(22) Date of filing: 07.11.2011
(51) Int. Cl.: A61M 5/32, A61M 5/178

(54) **SAFETY NEEDLE WITH COVERED TIP AND SIDES**

(71) Applicant: Medikit Co., Ltd., Bunkyo-ku Tokyo 1130034 (JP)
(72) Inventor: NAKAJIMA, Hiroaki, Tokyo 113-0034 (JP); KUROKAWA, Keisuke, Tokyo 113-0034 (JP)
(74) Representative: Kreutzer, Ulrich
(86) International application number: PCT/JP2011/075559
(87) International publication number: WO 2013/069075

(57) **Abstract**

A medical safety needle is comprised of a needle body having a needlepoint and a side face; a flexible sheath capable of fitting on the side face and so dimensioned as to hide the side face; and a slider slidably fitting on the needle body and the sheath so as to be movable from a first position to expose the needlepoint to a second position to hide the needlepoint and so dimensioned as to rub on the sheath so as to fit the sheath onto the needle body when the slider moves from the first position to the second position.

## Description

### BACKGROUND OF THE INVENTION

### FIELD OF THE INVENTION

The present invention relates to a needle for medical use and in particular relates to a safety needle, a needlepoint and a side face of which will be covered after piercing.

### DESCRIPTION OF THE RELATED ART

In order to introduce medicinal solution or a catheter into a patient's body, various needles for medical use have been used. After use, blood or any body fluid adheres on the needles for medical use and is sometimes contaminated with viruses such as HIV or hepatitis virus. The needles in medical use are potentially suspected to mediate infectious diseases.

There are proposed instruments in which points of needles will be, after use, covered for the purpose of prevention of infection.

The Patent Literatures 1-3 disclose related arts.

### [Citation List]

### [Patent Literature]

[PTL 1]: Published International Patent Application WO 90/06142
[PTL 2]: European Patent Application EP 1 935 447 A1
[PTL 3]: European Patent Application EP 2 338 554 A1

### SUMMARY OF THE INVENTION

Some of the aforementioned instruments hide needlepoints of needles but side faces of the needles are left exposed. These instruments successfully prevents infection caused by injury but are not effective against risks in that unspecified persons touch fluid adhering on the needles, in particular on these side faces. The other instruments may hide side faces of needles as well as needlepoints but cannot, in general, prevent fluid from flowing out along side faces of the needles or inner surfaces of the instruments. The present inventors had found out potential problems here and reached a safety needle that hides a side face as well as a needlepoint of its needle to retain fluid adhering to the needle, thereby the safety needle could solve potential problems that such fluid may give rise to.

An aspect of the present invention, a medical safety needle is comprised of a needle body having a needlepoint and a side face; a flexible sheath capable of fitting on the side face and so dimensioned as to hide the side face; and a slider slidably fitting on the needle body and the sheath so as to be movable from a first position to expose the needlepoint to a second position to hide the needlepoint and so dimensioned as to rub on the sheath so as to fit the sheath onto the needle body when the slider moves from the first position to the second position.

### BRIEF DESCRIPTION OF DRAWINGS

Fig. 1 is a schematic perspective view of a safety needle according to an embodiment of the present invention.
Fig. 2A is a schematic longitudinal sectional view of the safety needle before use.
Fig. 2B is a schematic longitudinal sectional view of the safety needle, which depicts a state where a slider is moved.
Fig. 2C is a schematic longitudinal sectional view of the safety needle, which depicts a state where a sheath fits on the needle.
Fig. 3A is a schematic cross sectional view of a sheath in accordance with a first example.
Fig. 3B is a schematic cross sectional view of a sheath in accordance with a second example.
Fig. 3C is a schematic cross sectional view of a sheath in accordance with a third example.
Fig. 3D is a schematic cross sectional view of a sheath in accordance with a fourth example.
Fig. 3E is a schematic cross sectional view of a sheath in accordance with a fifth example.
Fig. 4A is a schematic longitudinal sectional view of a sheath without a cap body.
Fig. 4B is a schematic longitudinal sectional view of a sheath with a cap body in accordance with an example.
Fig. 4C is a schematic longitudinal sectional view of a sheath with a cap body in accordance with another example.
Fig. 4D is a schematic longitudinal sectional view of a sheath with a solid cap body.
Fig. 5A is a schematic elevational view of a sheath without a cap body.
Fig. 5B is a schematic elevational view of a sheath without a cap body in accordance with another example.
Fig. 5C is a schematic elevational view of a sheath with a cap body in accordance with one example.
Fig. 5D is a schematic elevational view of a sheath with a cap body in accordance with another example.
Fig. 5E is a schematic elevational view of a sheath having a structure for engagement.
Fig. 6 is a schematic enlarged longitudinal sectional view showing a relation among the needle body, the sheath and the slider in a state corresponding to that shown in Fig. 2B.
Fig. 7A is a schematic sectional view showing a slider comprising a shutter.
Fig. 7B is a schematic plan view of the slider seen from a needlepoint of the needle.
Fig. 7C is a schematic perspective view of the shutter.
Fig. 7D is a schematic sectional view showing a state where the shutter closes a through-hole of the slider.
Fig. 8A is a schematic cross sectional view of a slider with a shutter in accordance with a modified example.
Fig. 8B is a schematic cross sectional view showing a state where the shutter shown in Fig. 8A is retracted.
Fig. 9A is a schematic elevational view of a slider in accordance with another modified example.
Fig. 9B is a schematic elevational view showing a state where a shutter is retracted.
Fig. 10 is a schematic longitudinal sectional view of a shutter and a slider in accordance with still another modified example.
Fig. 11 is a schematic longitudinal sectional view illustrating in detail a relation among the needle body, the sheath and the slider in accordance with the modified example.
Fig. 12 is a schematic longitudinal sectional view illustrating in detail a relation among the needle body, the sheath and the slider in accordance with still another modified example.
Fig. 13 is a schematic perspective view of the safety needle according to another embodiment.
Fig. 14A is a schematic longitudinal sectional view illustrating in detail a relation among the needle body, the sheath and the slider of the safety needle shown in Fig. 13.
Fig. 14B is a schematic longitudinal sectional view illustrating a state where a shutter shown in Fig. 14A is retracted.
Fig. 15 is a schematic perspective view of a safety needle according to still another embodiment.
Fig. 16A is a schematic cross sectional view showing one example of a sheath applied to the safety needle shown in Fig. 15.
Fig. 16B is a schematic cross sectional view showing a sheath of another example.
Fig. 16C is a schematic cross sectional view showing a sheath of still another example.
Fig. 17 is a schematic perspective view of a safety needle according to another embodiment.
Fig. 18 is a schematic elevational view of a safety needle according to still another embodiment, which shows a partial cross section.
Fig. 19A is a schematic elevational sectional view of a safety needle according to still another embodiment.
Fig. 19B is a schematic elevational sectional view of the safety needle, which depicts a state where a slider is moved.
Fig. 19C is a schematic longitudinal sectional view of the safety needle, which depicts a state where a needlepoint is hidden.

### DETAILED DESCRIPTION OF EXEMPLARY EMBODIMENTS

Exemplary embodiments of the present invention will be described hereinafter with reference to the appended drawings. Any of safety needles according to these embodiments is usable for the purpose of introduction of fluid such as medicinal solution or a tube such as a catheter to a patient's body. Application is not limited to human beings but also to any creatures of course, and to any nonliving thing as well. Whereas the embodiments described later relate to an application as a catheter introducer, they may not be limited to this of course but also to any applications such as a syringe.

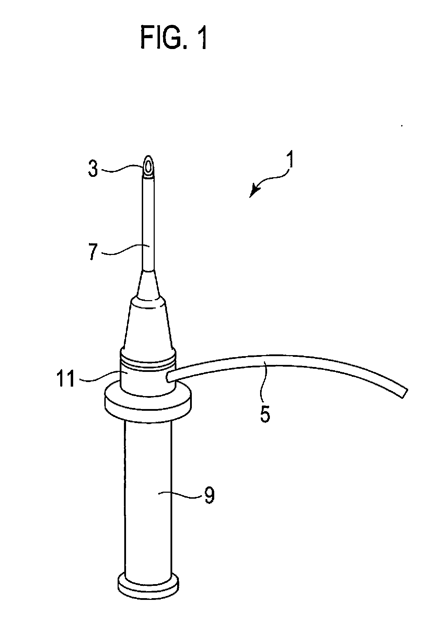

Referring to Fig. 1, a safety needle 1 of an embodiment is comprised of a needle body 3 insertable into a patient's body, a sheath 5 for fitting on the needle body 3 and hiding its needlepoint and its side face, a catheter 7 fitting on the needle body 3, a handle 9 to which the safety needle 1 is fixed, and a slider 11 movable toward the needlepoint so as to make the sheath 5 fit on the needle body 3. In a case where the safety needle 1 is used as a syringe, the catheter 7 is omitted and the handle 9 is structured as a cylinder for injecting or extracting liquid.

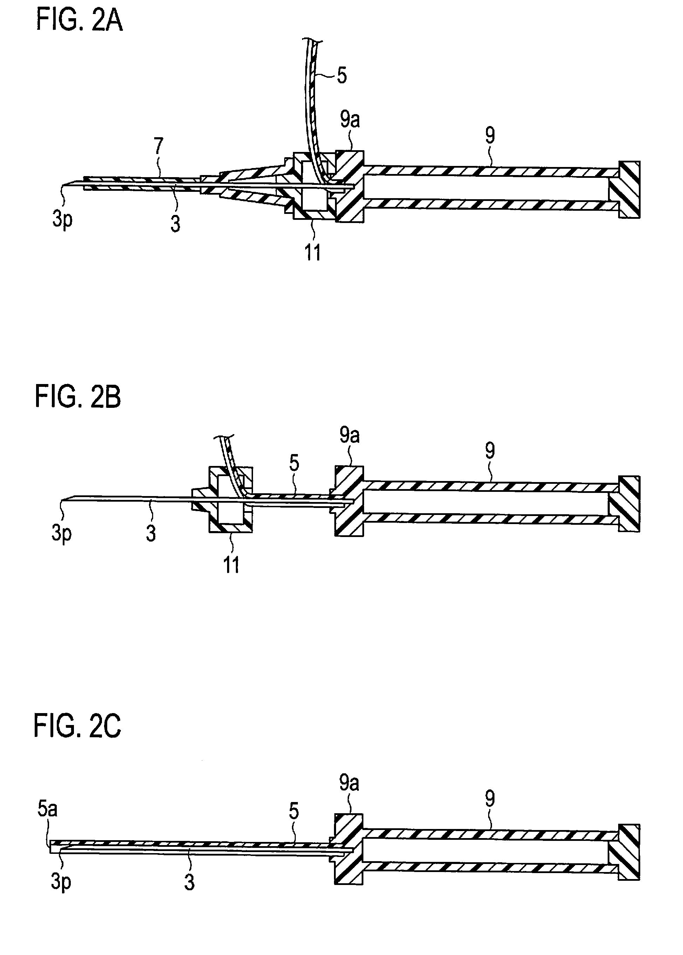

Referring to Fig. 2A as well as Fig. 1, the needle body 3 may be either hollow or solid and is comprised of a needlepoint 3p for piercing a patient's body, and a side face. At a proximal end opposed to the needlepoint 3p, the needle body 3 is fixed to a shoulder portion 9a of the handle 9. Fixation is made by any proper means such as press-fitting. The proximal end of the needle body 3 may project into the handle 9 or buried in the shoulder portion 9a. In a state before use, the slider 11 fits on the needle body 3 and, in a case where the catheter is used, the catheter 7 fits thereon and the side face is substantially thoroughly covered by them. A rear end of the catheter 7 may fit on the slider 11. The catheter 7 is, after the needle pierces the patient's body, left on the patient's body.

The slider 11 is, in a state shown in Fig. 2A, at a first position where the slider is in contact with the shoulder portion 9a of the handle 9, and then the needlepoint 3p is exposed. The slider 11 is, as shown in Fig. 2B, movable toward the needlepoint 3p and then rubs on the sheath 5 so as to fit the sheath 5 onto the needle body 3. When the slider 11 further moves to a second position where the slider 11 hides the needlepoint 3p, the sheath 5 fits on and hides the needlepoint 3p and the side face totally. Alternatively the sheath 5 may not fit on a limited part of the needle body 3. A user may thereafter move the slider 11 further to separate it from the needle body 3 as shown in Fig. 2C.

Meanwhile, throughout the present description and appended claims, the term "hide" means to substantially prevent contact from the exterior into the interior.

The sheath 5 is of a flexible material and, at or near its proximal end, fits on the needle body 3 and is together therewith fixed to the shoulder portion 9a of the handle 9. In a state before use, any part except the proximal end or its vicinity is made apart from the needle body 3 and is led out through a side face of the slider 11 as shown in Fig. 2A for instance. The sheath 5 is of a shape capable of fitting on the needle body 3 as described later and is so dimensioned as to substantially hide the needlepoint 3p and the side face of the needle in total when it fits on the needle body 3. As the flexible material, polypropylene, polyvinyl chloride, polyurethane, polyethylene, nylon, and fluoropolymers are exemplified but not limiting. A very thin and pliable metal for example may be applied to the sheath 5.

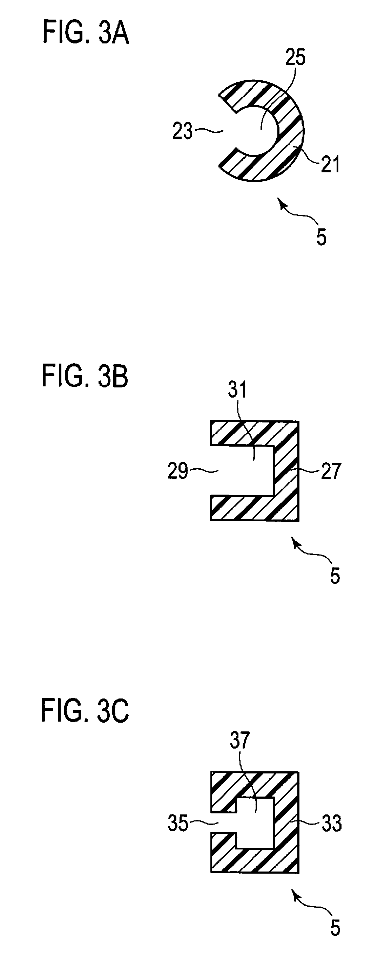

Referring to FIGs. 3A through 3E, the sheath 5 has a shape adapted for fitting on the needle body 3.

The sheath 5 may be of a hollow cylinder-like shape as shown in Fig. 3A for instance and its cylinder-like wall 21 has a slot 23 elongated along its longitudinal direction to receive the needle and a cavity 25 enclosed by the wall 21, so that the needle is received in the cavity 25 to fit therein. More specifically, the wall 21 hides the side face of the needle. The slot 23 may be either slightly open as shown in the drawings or closed before receiving the needle. In accordance with such a shape, after the sheath 5 and the needle body 3 fit together, the needle and the sheath come into tight contact, thereby effectively preventing the needle once housed in the cavity 25 from sticking out.

Instead of the cylinder, a rectangular shape elongated in the longitudinal direction of the sheath 5 may be applied thereto. A rectangular wall 27 has a slot 29 as shown in Fig. 3B for instance and the needle fits in a rectangular cavity 31 formed therein. This shape is advantageous as twisting is least likely. While a narrow clearance may be held between the needle body 3 and the wall 27, any liquid such as blood or any body fluid adhering on the side face of the needle is retained in the clearance. As such liquid, by viscosity and surface tension of itself, tends to stay there, the clearance is more advantageous in prevention of liquid leakage.

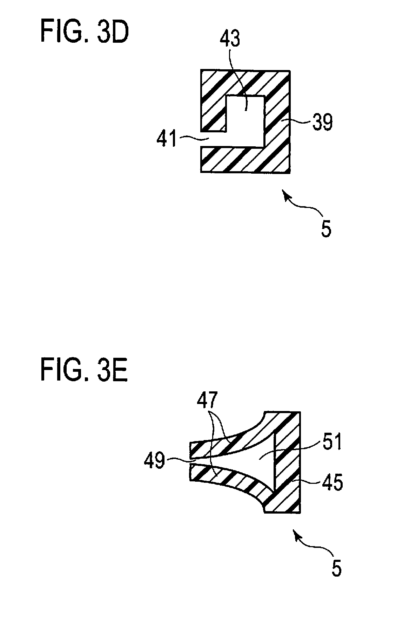

Further, as shown in Fig. 3C, the wall 33 may arch out in directions narrowing the slot 35. The needle gets through the narrowed slot 35 to fit in a cavity 37 therein. This shape effectively prevents the needle once housed in the cavity 37 from sticking out. Or, as shown in Fig. 3D, only one of edges of the wall 33 may arch out. The needle gets through the narrowed slot 41 to fit in a cavity 43 therein. This shape also effectively prevents the needle once housed in the cavity 43 from sticking out.

Or, as shown in Fig. 3E, the sheath 5 may be constituted of a wall 45 elongated in its longitudinal direction and a pair of valve-like bodies 47 projecting from both lateral edges of the wall toward each other. The needle gets through a narrowed slot 49 held between the valve-like bodies 47 to fit in a cavity 51 therein. As the valve-like bodies 47 are urged toward a direction closing the slot 49, this effectively prevents the needle once housed in the cavity 51 from sticking out.

The shapes adapted for fitting on the needle body 3 as described above may be limited to part close to the needlepoint 3p and its vicinity. Further, depending on cases, the sheath 5 throughout the length may be strip-like or wire-like for instance, instead of any of the aforementioned shapes.

The aforementioned clearance may be held in not only the sheath shown in Fig. 3B but also any other sheath such as the cylindrical sheath shown in Fig. 3A for instance. The clearance is preferably held wider in light of a greater capacity for retaining the liquid. Thus the clearance may be 0.01 - 1 mm in width for example.

The sheath 5, at least its internal surface, may be processed with a hydrophilicity treatment by any known technique such as a chemical modification, a plasma treatment or such. Blood will adhere on the needlepoint 3p and the side face after use, the hydrophilicity-treated internal surface is advantageous in retaining the blood between the needle body 3 and the sheath 5. Alternatively, a hydrophobicity treatment may be applied thereto.

Instead of, or in addition to, the hydrophilicity treatment, absorbency may be given to the sheath 5. The sheath 5 may be lined with an absorbent fibrous layer for instance, or may be made of an absorbent material. Such structures are also advantageous in retaining blood.

In either case, the sheath 5 does not merely hide the needlepoint 3p and the side face of the needle but also, after hiding, effectively prevents liquid such as blood or body fluid from flowing out.

The exterior of the sheath 5 may be processed with a hydrophobicity treatment or a hydrophilicity treatment. Or, the sheath 5 may be made of a hydrophobic or hydrophilic material.

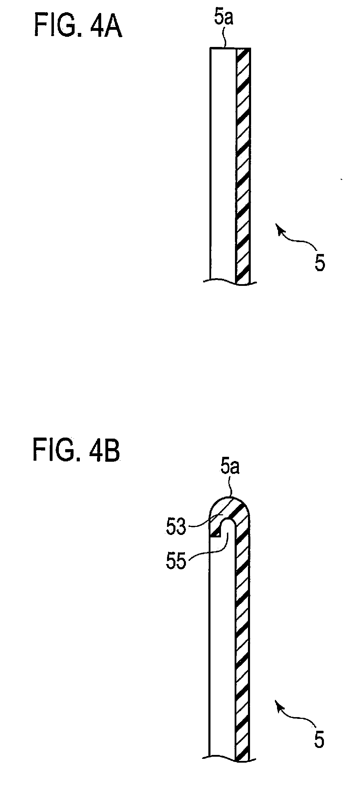

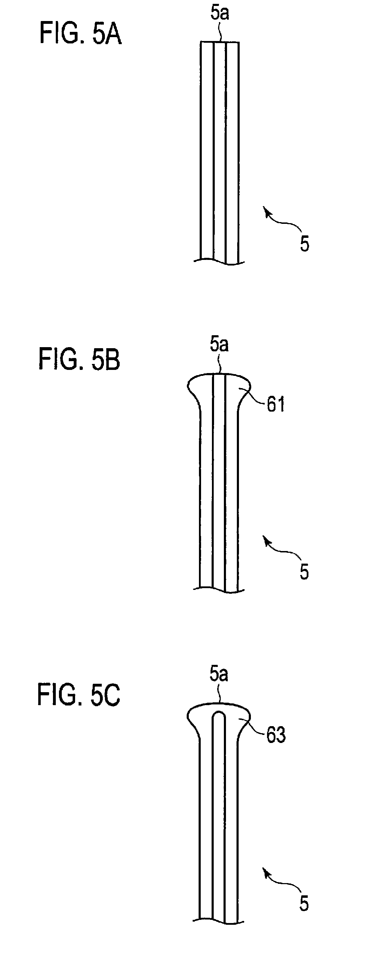

Referring to FIGs. 4A through 4D and FIGs. 5A through 5E, some structures applied to a tip 5a of the sheath 5 will be described hereinafter.

As shown in Fig. 4A and Fig. 5A, the sheath 5 may be opened toward the tip 5a. Alternatively, as shown in Fig. 4B, the tip 5a may be comprised of a cap body 53 closing the opening. The cap body 53 prevents blood from trickling down from the needlepoint 3p and also effectively prevents the sheath 5 from displacing from the needlepoint 3p as the needlepoint 3p gets caught in the cap body 53. In order to enhance these effects, a hollow 55 may be held in the cap body 53.

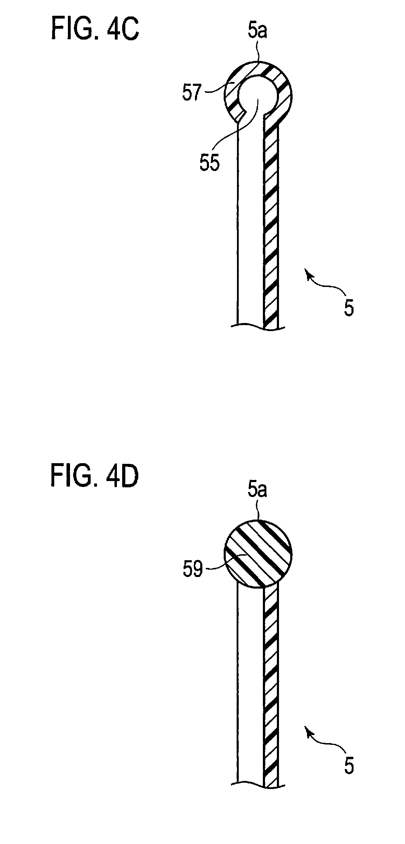

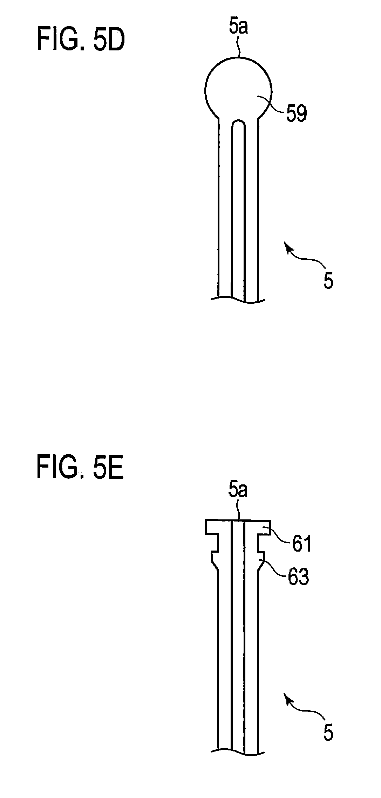

Alternatively, as shown in Fig. 4C and Fig. 5D, a cap body 57 may bulge out as a ball-like shape. The bulging cap body 57 can give a greater capacity to the hollow 55 therein for retaining blood or body fluid. A ball-like cap body 59 may be, of course, alternatively solid as shown in Fig. 4D. As will be described later in detail, the ball-like cap body latches on the slider 11, thereby preventing it from falling out, or may be used for automatically retracting a shutter described later.

Alternatively, as shown in Fig. 5B, a cap body 61 may be opened toward the tip 5a as well as bulge outward. In a bulging cap body 63, a tip 5a may be, of course, closed as shown in Fig. 5C.

Still alternatively, as shown in Fig. 5E, the tip 5a may be comprised of projections 61,63 as shown in Fig. 5E for the purpose of latching on the slider 11 or the shutter for example.

The aforementioned cap bodies may be formed by heating and thereby deforming the tips of the sheaths. Alternatively, a cap body may be in advance formed independent of the sheath and then bonded to the sheath by any means such as thermal or ultrasonic bonding. Or, knotting the sheath may be applicable to formation of the bulging tip. Crimping a ring of a metal or any other material onto the sheath may be still applicable.

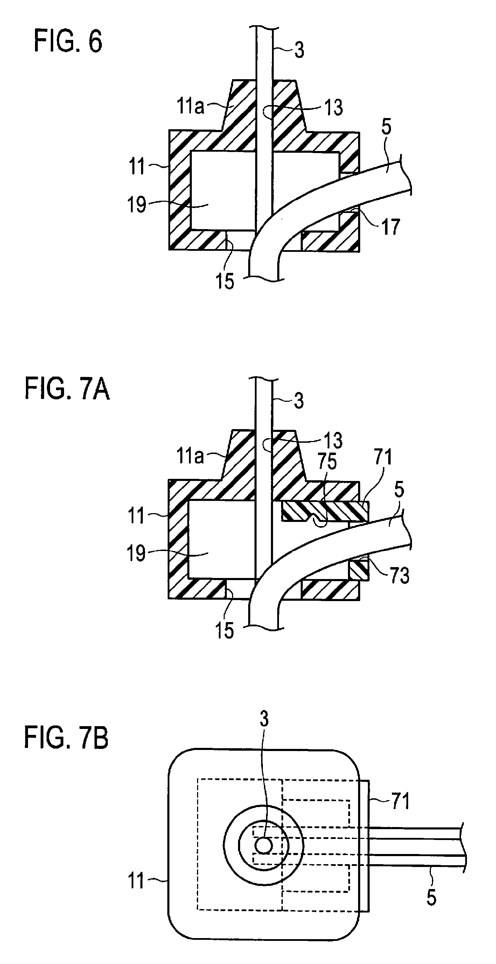

Referring to FIGs. 6 through 12, some structures applied to the slider 11 will be described hereinafter. The slider 11 may be formed of, but not limited to, any proper resin such as polycarbonate.

Referring to Fig. 6, the slider 11 according to one embodiment has a head portion 11a projecting upward from its main body, a through-hole 13 penetrating the head portion 11a, a through-hole 15 penetrating a lower portion of the main body, a through-hole 17 penetrating a side face of the main body, and a cavity 19 held in the main body and being in communication with the through-holes.

The through-hole 13 at the top is relatively small in diameter and fits on the needle body 3. As the head portion 11 a is elongated upward, the through-hole 13 is also relatively long and therefore fits on a relatively long range of the needle body 3. The slider 11, as being guided by the through-hole 13, can smoothly slide on the needle body 3 without oscillation. The head portion 11 a, as being understood from Fig. 2A, fits in and thus supports the catheter 7.

The through-hole 15 at the bottom and the through-hole 17 at the side are greater in diameter than the through-hole 13 at the top so as to allow passage of the sheath 5. The sheath 5, in the vicinity of the through-hole 15 at the bottom, gets apart from the needle body 3 and is led out of the slider 11 through the through-hole 17 at the side.

As being understood from Fig. 6, the through-hole 15 and the through-hole 17 steadily butt against the sheath 5 and thus deflect and bias the sheath 5 toward the needle body 3. When the slider 11 moves toward the needlepoint as shown in Fig. 2B, the through-hole 15 and the through-hole 17 therefore make the sheath 5 fit on the needle body 3. More specifically, the slider 11 is so dimensioned as to rub on the sheath 5 so as to fit the sheath 5 onto the needle body 3 when moving.

In the meantime, the shoulder portion 9a of the handle 9 and the lower portion of the slider 11 preferably have a structure adapted for mutually engaging and retaining the slider at its initial position shown in Fig. 2A. The shoulder portion 9a of the handle 9 may be for example comprised of a projection fitting in the through-hole 15. Alternatively, the periphery of the through-hole 15 may be comprised of a projection elongated downward and the shoulder portion 9a of the handle 9 may be comprised of a depression corresponding thereto.

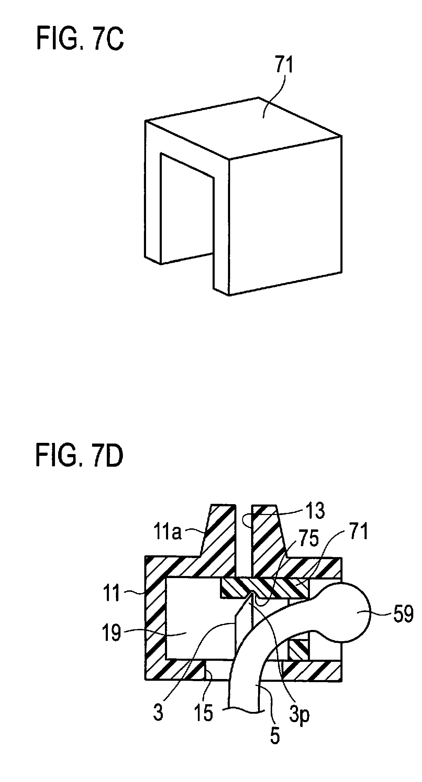

Referring to FIGs. 7A through 7D, the slider 11 may be comprised of a shutter 71 fitting in the slider 11 and retractable laterally. The shutter 71 may, as shown in the drawings, project slightly outward through the through-hole at the side, or may totally recede inward. The shutter 71 may be formed of, but not limited to, any proper resin such as polycarbonate. The shutter 71 is a rectangular box opened at its bottom at least for example and has a through-hole 73 on its side face. The sheath 5 butts against the through-hole 73 of the shutter 71.

As being understood from Fig. 7A and Fig. 7B, the shutter 71 is initially at a position not interfering the needle body 3 and its end may project slightly from the side face of the slider 11. When the slider 11 moves toward the needlepoint 3p and then the needlepoint 3p gets down into the slider 11, the cap body 59 of the sheath 5 gets caught on the through-hole 73 of the shutter 71. When the slider 11 is attempted to further advance, the caught cap body 59 draws the shutter 71 into the slider 11 so that the shutter 71 closes the through-hole 13 at the top of the slider 11. The needlepoint 3p is prevented from projecting out of the slider 11 and the blood will not leak out through the through-hole 13.

In order to further effectively prevent blood leakage, the shutter 71 may be comprised of an absorbent member or formed of an absorbent material.

The shutter 71 may be comprised of a depression 75 engageable with the needlepoint 3p so as not to get in motion again. The slider 11 may be comprised of a bias means such as a spring in order to draw the shutter 71 therein. In this case, as the bias means retracts the shutter 71 as soon as the needlepoint 3p gets down into the through-hole 13, the sheath 5 is not required to have the cap body.

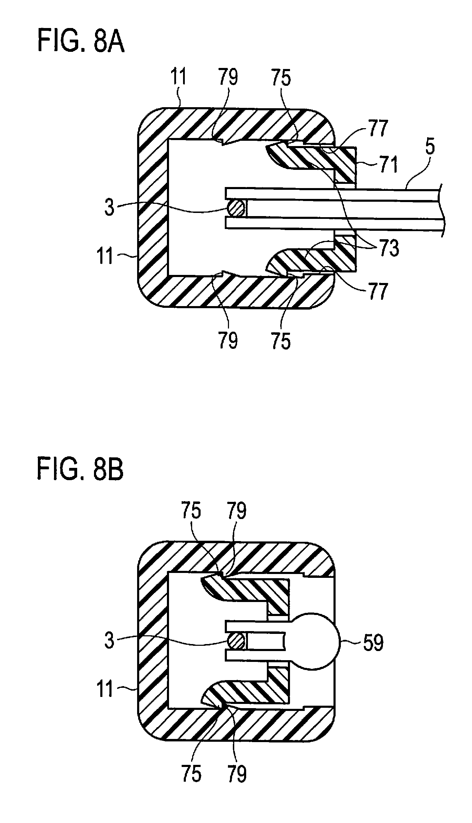

The shutter 71 may be comprised of a proper latching means for the purpose of being locked at a specific position relative to the slider 11. The shutter 71 in an example shown in FIGs. 8A and 8B for example is comprised of resilient lances 73 and detents 75 respectively projecting outward from ends thereof. The slider 11 is correspondingly, at its internal surfaces, comprised of locking detents 77,79.

As the locking detents 77 close to the side face latch on the detents 75, the shutter 71 is locked at the initial position. When the shutter 71 retracts therein, the lances 73 deflect inward so that the detents 75 get over and thus latch on the locking detents 79. Then the lances 73 cause a clicking noise and a clicking feeling, which inform the user of that the shutter 71 reaches the retracted position. By the engagement of the detents 75 and the locking detents 79, the shutter 71 is kept at the retracted position.

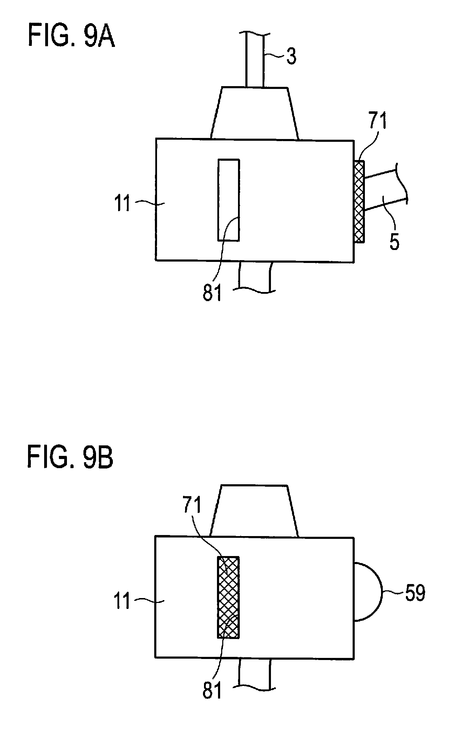

Referring to Fig. 9A and Fig. 9B, the slider 11 may be comprised of a window 81 for verification of the position of the shutter 71. When the shutter 71 is at the initial position, nothing comes in sight through the window 81 as shown in Fig. 9a, but when the shutter 71 is retracted and set in place, the shutter 71 comes in sight through the window 81 as shown in Fig. 9B. The shutter 71 may be colored for ease of verification.

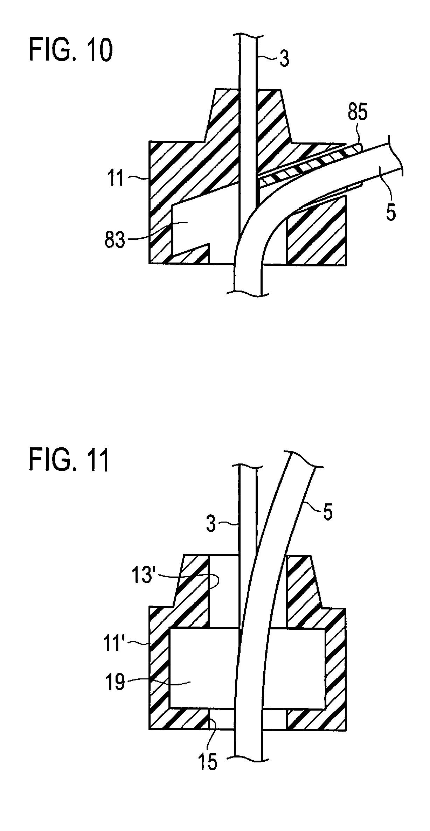

While the shutter is, in the above descriptions, explained to be of a shape moving horizontally, it may be modified to be of a shape moving obliquely. More specifically, as shown in Fig. 10, the slider 11 may have an oblique cavity 83 and the shutter 85 may be of a corresponding shape. The direction where the shutter moves is more consistent with the direction where the sheath 5 runs, thereby easing motion of the shutter 85.

The sheath 5 may not be necessarily led out through the side face of the slider. Referring to Fig. 11, a through-hole 13' is formed relatively large in diameter so as to allow passage of the sheath 5, through which the sheath 5 is led out of the slider 11. This structure also causes no difficulty in motion of the slider 11' to fit the sheath 5 onto the needle body 3.

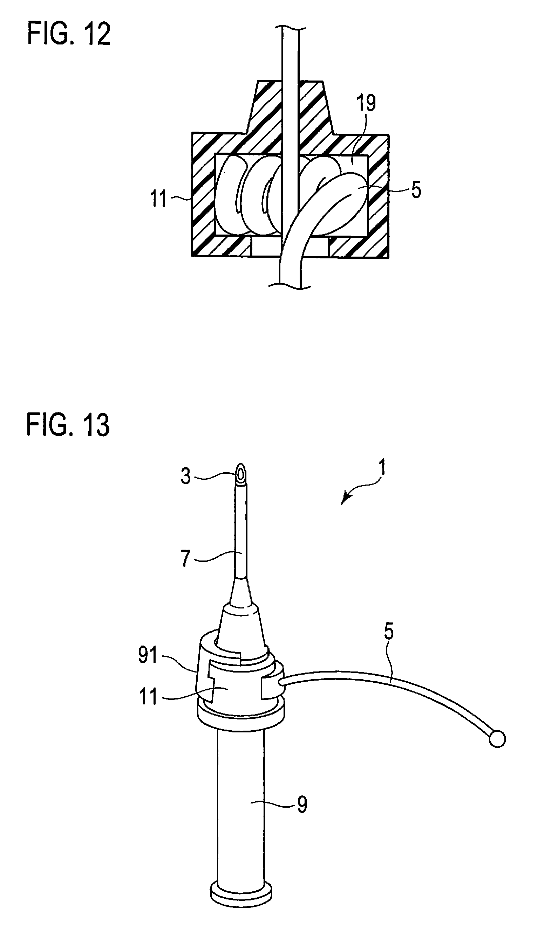

As well, the sheath 5 may not be necessarily led out of the slider. Referring to Fig. 12, the slider 11 may be formed relatively large and the whole of the sheath 5 may be housed in the cavity 19 therein. This structure also causes no difficulty in motion of the slider 11 to fit the sheath 5 onto the needle body 3.

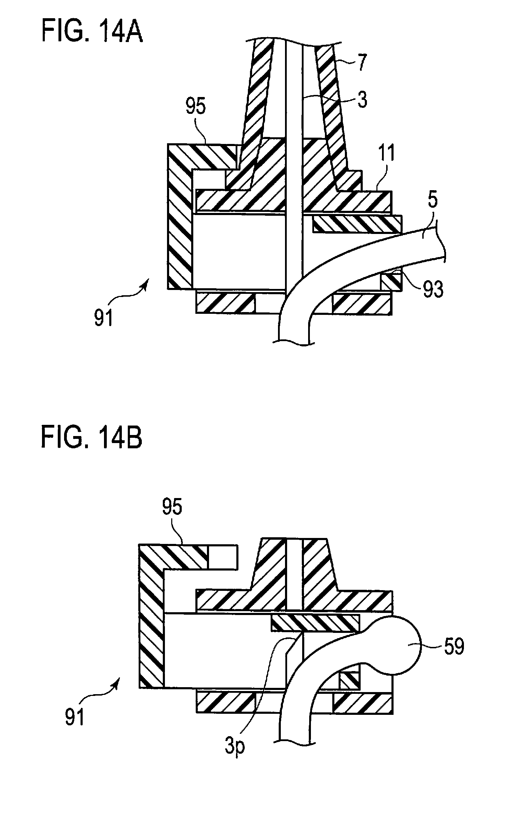

Referring to FIGs. 13 through 14B, the safety needle 1 may be further comprised of a locking member 91 for temporarily securing the catheter 7 to the slider 11. The locking member 91 preferably, as shown in Fig. 14A, fits in the interior of the slider 11 and is comprised of a through-hole 93 on one end thereof, thereby acting as the shutter described above does. The locking member 91 is, at the opposite end, elongated upward and thus forms a latch 95 latching on the proximal portion of the catheter 7.

When the slider 11 is at the initial first position or even at a position slightly advanced therefrom, the latch 95 keeps latching on the catheter 7, thereby uniting the catheter 7 to the slider 11.

When the slider 11 is advanced to the second position to hide the needlepoint 3p, the cap body 59 of the sheath 5 gets caught in the through-hole 93 so that the locking member 91 moves leftward as shown in Fig. 14B. The locking member 91 closes the through-hole at the top of the slider 11 as well as the catheter 7 is released from the latch 95 and thereby removed from the safety needle 1 automatically.

As being understood from the above description, as the catheter 7 is, before use of the safety needle 1, locked by the latch 95, the catheter 7 is not displaced from the needle body 3 unintentionally. Even at the moment of forwarding the slider 11 after use of the safety needle 1, the catheter 7 keeps integral with the slider 11, thereby hiding the needlepoint 3p. When the slider 11 reaches the position to hide the needlepoint 3p, the catheter 7 eventually gets released from the latch 95. More specifically, during the series of manipulation, either the catheter 7 or the sheath 5 consistently keeps hiding the whole of the needle body 3. Anyone is prevented from unintentionally touching the needle body 3.

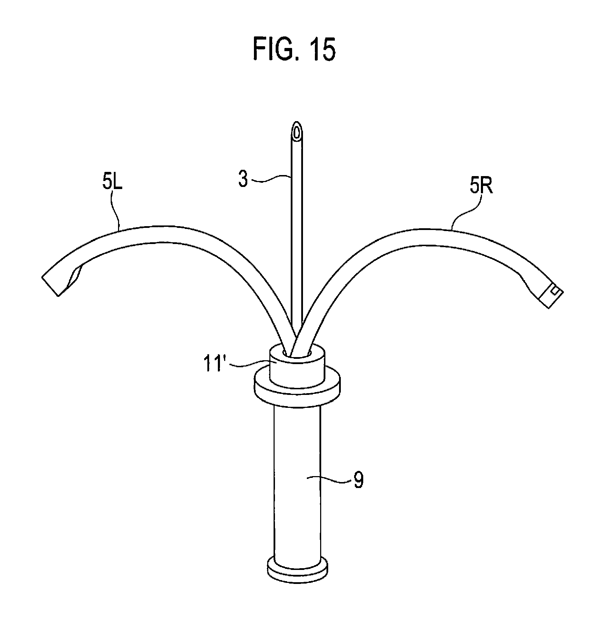

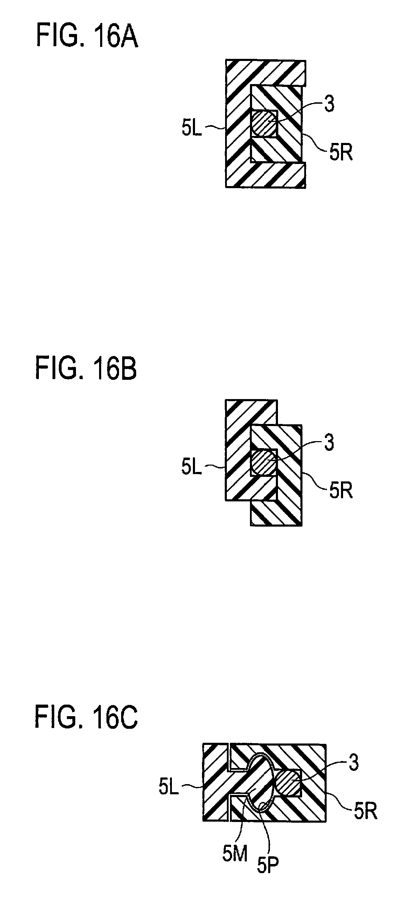

Further, the sheath may not be necessarily a single unitary member but, as shown in Fig. 15, sheaths 5L,5R mating together are applicable thereto. The sheath 5L may be as shown in Fig. 16A so configured as to fit on and enclose the sheath 5R fitting on the needle body 3. Alternatively they may be so configured that the sheath 5L, the needle body 3 and the sheath 5R cooperatively and mutually mate together as shown in Fig. 16B. Still alternatively, as shown in Fig. 16C, the sheath 5L may be comprised of a projection 5M for fitting and the sheath 5R may be comprised of a depression 5P receiving it, thereby mating together. Or, any adhesion agent or such is applicable for the purpose of securing the sheath 5L,5R together. In any case, as the sheaths thoroughly enclose the periphery of the needle, the needle is hidden more effectively.

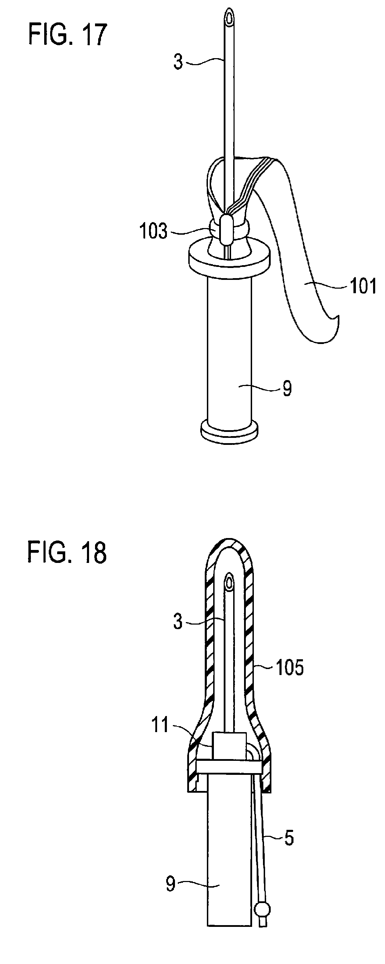

Alternatively, as shown in Fig. 17, a sheath 101 may be so configured as to be a single unitary member but be capable of enclosing the whole of the needle body 3. Both lateral edges of the sheath 101 have a structure for mating together. A zip fastener (so-called "zipper") can be exemplified as such a structure. When moving a slider 103 toward the needlepoint, both edges are made to mate together, thereby the sheath 101 encloses the whole of the needle body 3. As the sheath thoroughly encloses the periphery of the needle, the needle is more effectively hidden.

In any of these cases in the above description in common, the safety needle 1 may be, as shown in Fig. 18, comprised of a cover 105 for protecting the needle body 3 and the catheter 7. The cover 105 preferably has a cut through which the sheath 5 is led out, which is so structured as to press and bend the sheath 5 downward. The handle 9 may be also comprised of a cut for receiving the sheath 5. The needlepoint 3p is, before use, effectively protected as well as oscillation of the sheath 5 is effectively prevented, thereby this structure is advantageous in delivery of the safety needle 1.

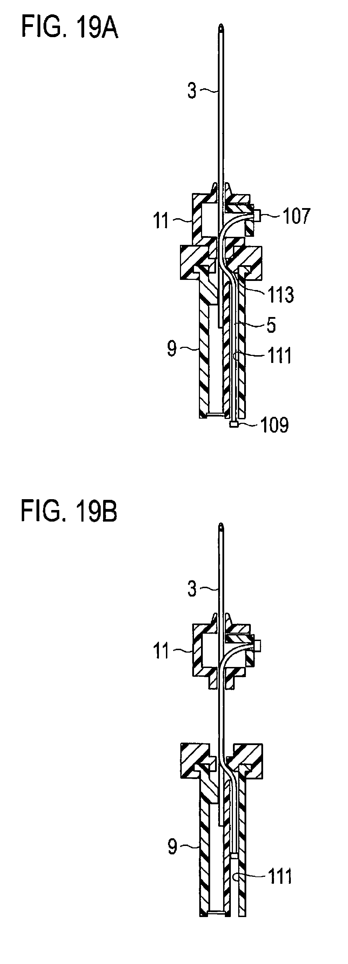

To house the sheath 5, the handle 9 or any other member may be used. FIGs. 19A through 19C show examples for housing the sheath 5.

Referring mainly to Fig. 19A that depicts a state before use, the slider 11 is at a first position where the slider 11 is in contact with the shoulder portion of the handle 9, and a proximal end 109 of the sheath 5 is not fixed to the handle 9 and retracted into a housing chamber 111 through an opening 113 provided on the handle 9. A proximal end of the housing chamber 111 may be either opened or closed. In a case being opened, the proximal end 109 may be exposed to the exterior or retracted in the interior. In any case, in a state before use, the majority of the sheath 5 is housed in the housing chamber 111. In the drawing, the sheath 5 is substantially straightened but may be curved or form a spiral.

The sheath 5 is, at its both ends 107,109, comprised of a bulge portion or a cap body for latching. Alternatively, at either or both of the ends 107,109, its interior hollow may be closed. As the closed hollow latches on the needle body 3, it may act as the bulge portion or the cap body does.

The sheath 5, near the through-hole at the bottom of the slider 11, fits on the needle body 3 and, just above it, gets apart from the needle body 3 and is drawn out through the side face of the slider 11 to the exterior. The tip end 107 of the sheath 5 may initially latch on the side face of the slider 11 and may, in a case where the slider 11 comprises the shutter, initially latch on the side face of the shutter.

Referring to Fig. 19B, when the slider 11 moves toward the needlepoint 3p, the slider 11 rubs on the sheath 5 so as to fit the sheath 5 onto the needle body 3 and also draws the sheath 5 out of the housing chamber 111. The opening 113 of the handle 9 then presses the sheath 5 onto the needle body 3 to retain the fitting of the sheath 5. Therefore, at the trace where the slider 11 passes and above the opening 113, the sheath 5 fits on the needle body 3.

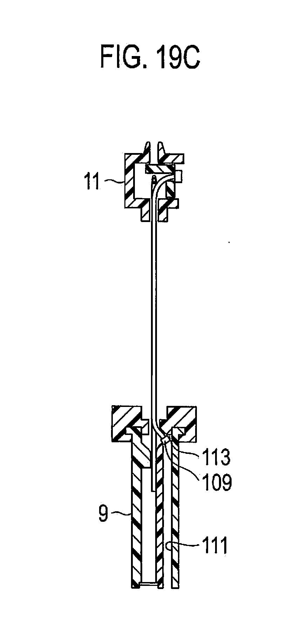

When the slider 11 moves to the second position shown in Fig. 19C where the slider 11 hides the needlepoint 3p, the sheath 5 substantially thoroughly fits on and then hides the needle body 3. As the tip end 107 latches on the through-hole and the proximal end 109 latches on the opening 113, the slider 11 is there prevented from displacing off. Alternatively, the closed proximal end 109 gets caught by the needle body 3, the slider 11 is prevented form displacing off. Thereby the needlepoint 3p is prevented from being exposed out. In a case where the slider 11 is comprised of the shutter, the shutter is driven by the tip end 107 to close the through-hole at the top of the slider 11.

In accordance with the present embodiments, as the sheath 5 does not project sideways, a user readily manipulates the safety needle 1.

Although the invention has been described above by reference to certain embodiments of the invention, the invention is not limited to the embodiments described above. Modifications and variations of the embodiments described above will occur to those skilled in the art, in light of the above teachings.

### INDUSTRIAL APPLICABILITY

A safety needle capable of hiding a needlepoint as well as a side face of a needle is provided.

## Claims

1. A medical safety needle comprising:
a needle body having a needlepoint and a side face;
a flexible sheath capable of fitting on the side face and so dimensioned as to hide the side face; and
a slider slidably fitting on the needle body and the sheath so as to be movable from a first position to expose the needlepoint to a second position to hide the needlepoint and so dimensioned as to rub on the sheath so as to fit the sheath onto the needle body when the slider moves from the first position to the second position.

2. The medical safety needle of claim 1, wherein the sheath is further so dimensioned as to hide the needlepoint.

3. The medical safety needle of claim 1, wherein the slider comprises a first through-hole configured to rub on the sheath and a second through-hole, and the needle body penetrates the first through-hole and the second through-hole.

4. The medical safety needle of claim 3, wherein the slider comprises a third through-hole and a tip of the sheath is led out of the third through-hole when the slider is at the first position.

5. The medical safety needle of claim 4, further comprising:
a shutter fitting with the slider to be retractable in an interior of the slider to close the second through-hole.

6. The medical safety needle of claim 5, wherein the sheath comprises a cap body engageable with the shutter to retract the shutter.

7. The medical safety needle of claim 5, wherein the shutter comprises an engaging detent.

8. The medical safety needle of claim 1, wherein the sheath comprises a wall configured to hide the side face, the wall defining a hollow so dimensioned as to accommodate the needle body.

9. The medical safety needle of claim 8, wherein the wall is so dimensioned as to have a clearance capable of retaining liquid between the wall and the side face.

10. The medical safety needle of claim 1, wherein the sheath comprises a cap body for hiding the needlepoint.

11. The medical safety needle of claim 1, further comprising:
a housing chamber for housing the sheath.

12. The medical safety needle of claim 11, further comprising:
a handle having the housing chamber in the handle and configured to rub on the sheath.
